(19) Europäisches Patentamt
European Patent Office
Office européen des brevets

(11) **EP 4 144 766 A1**

(12) **EUROPEAN PATENT APPLICATION**
published in accordance with Art. 153(4) EPC

(43) Date of publication:
**08.03.2023 Bulletin 2023/10**

(51) International Patent Classification (IPC):
***C08B 37/10*** *(2006.01)*

(21) Application number: **20934039.7**

(52) Cooperative Patent Classification (CPC):
**C08B 37/0075**

(22) Date of filing: **10.11.2020**

(86) International application number:
**PCT/ES2020/070695**

(87) International publication number:
**WO 2021/219907 (04.11.2021 Gazette 2021/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.04.2020 PCT/ES2020/070271**

(71) Applicant: **Laboratorios Farmacéuticos Rovi, S.A.**
**28037 Madrid (ES)**

(72) Inventors:
• **FRANCO RODRÍGUEZ, Guillermo**
  **28037 Madrid (ES)**
• **GUTIERRO ADURIZ, Ibon**
  **28037 Madrid (ES)**

(74) Representative: **Elzaburu S.L.P.**
**Edificio Torre de Cristal**
**Paseo de la Castellana 259 C, planta 28**
**28046 Madrid (ES)**

(54) **METHOD FOR OBTAINING LOW-MOLECULAR-WEIGHT HEPARINS AND LOW-MOLECULAR-WEIGHT HEPARINS THEREBY OBTAINED**

(57) The invention relates to a method for obtaining low molecular weight heparins which exhibit high stability. The method includes the treatment of depolymerized heparin with $H_2O_2$ in a ratio of between 0.04 and 1.0 litres of $H_2O_2$ at 33 % w/v per kg of depolymerized heparin. The invention also relates to a heparin obtained by this method.

EP 4 144 766 A1

**Description**

**FIELD OF THE INVENTION**

The present invention relates to a process for the preparation of low molecular weight heparins (LMWH) and to the low molecular weight heparins obtained by that process.

**BACKGROUND OF THE INVENTION**

Heparin is a polysaccharide of the glycosaminoglycan family, formed by uronic acid (L-iduronic acid or D-glucuronic acid) and D-glucosamine, alternately bound. The L-iduronic acid may be 2-O-sulfated and the D-glucosamine may be N-sulfated and/or 6-O-sulfated, and to a lesser extent N-acetylated or 3-O-sulfated. The heparin is preferably used as a sodium salt but can also be used as a salt of other alkali or alkaline earth metals and is mainly used as an antithrombotic and anticoagulant drug.

Heparins can be classified according to their molecular weight into unfractionated heparin (UFH), LMWH and very low molecular weight heparin (VLMWH). LMWH and VLMWH are obtained by depolymerization of the original UFH molecule.

Several methods for the preparation of LMWH have been described in the prior art. One of them corresponds to alkaline depolymerization by a β-elimination mechanism.

EP0040144 describes a process for obtaining LMWH by a method comprising the steps of transalkylation of a heparin salt into benzethonium heparinate, esterification of the benzethonium heparinate with benzyl chloride, purification and obtaining the sodium salt of the benzyl ester of the heparin, depolymerization with sodium hydroxide with saponification of the ester and purification of the product.

EP1070503 describes a process for obtaining LMWH by a method comprising the steps of transalkylation of a heparin salt in benzalkonium heparinate, depolymerization in non-aqueous medium with Triton B and purification of the product.

EP2881404 describes a process for obtaining LMWH comprising a first transalkylation step, depolymerization with a phosphazene base or a guanidine-derived base and a final transalkylation.

There is a need for more reproducible and stable processes for obtaining LMWHs, especially LMWHs with greater stability.

**DESCRIPTION OF THE INVENTION**

The inventors of the present invention have found a method of preparing low molecular weight heparins (LMWH) which exhibit superior stability, while maintaining good anti-FXa and anti-FIIa activity. This method of preparation of LMWH comprises treating crude depolymerized heparin with $H_2O_2$ in a ratio of between 0.04 and 1.0 litres of $H_2O_2$ at 33 % w/v per kg of depolymerized heparin.

Therefore, a first aspect of the invention relates to a method for obtaining low molecular weight heparins with an average molecular weight of between 3 and 3.8 KDa, comprising the following steps:

a) preparing an aqueous solution of heparin sodium;
b) adding benzalkonium chloride to the solution of step a) to obtain benzalkonium heparinate;
c) dissolving the benzalkonium heparinate obtained in step b) in $Cl_2CH_2$, adding Triton B and maintaining a temperature between 20 and 40 ºC for 24 to 48 hours; and
d) carrying out at least two treatments with $H_2O_2$ of the depolymerized heparin obtained in step c) in a ratio of between 0.04 and 1.0 litres of $H_2O_2$ at 33 % w/v for each kg of depolymerized heparin in each treatment.

A second aspect of the invention relates to a low molecular weight heparin obtainable by the method of the invention.

It has been found that the obtained LMWHs exhibit a 1,6-anhydro residue content of between 1 and 15 %. Therefore, a third aspect of the invention relates to a low molecular weight heparin with an average molecular weight of between 3 and 3.8 kDa having a 1,6-anhydro residue content of between 1 and 15 % at the reducing terminus of its oligosaccharide chains.

The definitions and embodiments described for one aspect apply equally to all other aspects of the invention.

In the present invention "low molecular weight heparin" or "LMWH" is understood as defined in the document "Heparins, Low-Molecular-Mass monograph, 0828, European Pharmacopeia 9th Ed.", as the polysaccharide mixture obtained from heparin and having an average molecular weight of less than 8,000 Dalton and where at least 60 % of its total mass has a molecular weight of less than 8,000 Da. The average molecular weight of the LMWH of the invention has been determined by the method of the European Pharmacopeia (Ph. Eur. 9th Ed.).

In the present invention "1 ,6-anhydro residues" is understood as various chemical groups that are generated at the terminal positions of the LMWHs during the depolymerization process. Non-limiting examples of these groups are 2-sulfo-amino-1,6-anhydro-2-deoxy-β-D-glucopyranose (1,6-anhydroglucosamine) and 2-sulfo-amino-1,6-anhydro-2-deoxy-β-D-mannopyranose (1,6-anhydromannosamine). The amount of these residues in the LMWH is expressed as the percentage of oligosaccharide chains that have these types of residues at their reducing terminus.

The 1,6-anhydro-terminal content of LMWH can be obtained by the analytical method described in the Enoxaparin Sodium monograph, 1097, European Pharmacopeia 9th Ed, described under Identification B. In this method, the molecule is extensively depolymerized with a mixture of heparinases I, II and III, and the residues generated are separated and quantified by strong anion exchange chromatography (SAX-HPLC). The 1,6-anhydro content, for example, is determined according to the following formula:

$$\% \ 1,6\text{-anhydro} = Mw^{\bullet} (A_1 + A_2 + A_3)^{\bullet} \ 100 \ / \ \Sigma Mw_x^{\bullet} A_x$$

where:

- Mw, average molecular weight
- $Mw_x$ , molecular weight of derivative x (see table 1097-1 of Enoxaparin Sodium monograph, 1097, European Pharmacopeia 9th Ed)
- $A_x$, peak area of derivative x
- $A_1$, peak area of the 1 ,6-anhydro derivative ∆IS
- $A_2$, peak area of 1 ,6-anhydro derivative ∆IIS
- $A_3$ , peak area of the 1 ,6-anhydro ∆IS-IS derivative.

The ratio of 1,6-anhydroglucosamine and 1,6-anhydromannosamine residues in LMWH can be determined by Nuclear Magnetic Resonance (NMR), e.g. by $^1H$ $^{13}C$ HSQC. The ratio between the two residues can be determined by integrating the signals corresponding to each of these residues in the spectrum of $^1H$ $^{13}C$ HSQC.

The anti-FXa and anti-FIIa activity of the LMWH of the invention has been determined by the chromogenic method of the European Pharmacopoeia (Ph. Eur. 9th Edition, Monograph 0828) and expressed in international units per mg.

The degree of colouration of LMWH can be determined according to method II of the European Pharmacopoeia chapter 2.2.2.

The term "ambient temperature" refers to a temperature between 20 and 25 ᵒC.

As used herein, the term "approximately" means ± 10 % of the given value.

The LMWHs of the invention have an average molecular weight (Mw) of between 3 and 3.8 kDa, preferably between 3 and 3.6 kDa.

In one embodiment, the LMWH of the invention has a 1,6-anhydro residue content of between 1 and 15 % at the reducing terminus of its oligosaccharide chains; preferably between 2 and 13 %, more preferably between 4 and 11%.

Preferably, the molar ratio of the 1,6-anhydroglucosamine residues in the LMWH of the invention is greater than or equal to that of the 1,6 anhydromannosamine residues. In a particular embodiment, the molar ratio of 1,6-anhydroglucosamine residues:1,6 anhydromannosamine residues in the LMWH is between 1:1 and 3:1, preferably between 1:1 and 2.5:1 or between 1.05:1 and 2.5:1.

In a particular embodiment of the invention, the LMWH have an average molecular weight of between 3 and 3.8 kDa, a 1,6-anhydro residue content of between 1 and 15 % at the reducing terminus of their oligosaccharide chains, and a ratio of 1,6-anhydroglucosamine residues greater than or equal to that of 1,6-anhydromannosamine residues, preferably between 1:1 and 3:1.

Preferably, the LMWH of the invention exhibits an anti-FXa activity of between 80-120 IU/mg and an anti-FIIa activity of between 5-20 IU/mg. In a particular embodiment, it exhibits an anti-FXa activity of between 95-120 IU/mg and an anti-FIIa activity of between 10-20 IU/mg.

In a particular embodiment of the invention, the LMWH has an average molecular weight of between 3 and 3.8 kDa, a 1,6-anhydro residue content of between 1 and 15 % at the reducing terminus of their oligosaccharide chains, and a ratio of 1,6-anhydroglucosamine residues greater than or equal to that of 1,6 anhydromannosamine residues, preferably between 1:1 and 3:1, an anti-FXa activity of between 80-120 IU/mg and an anti-FIIa activity of between 5-20 IU/mg.

Preferably, the LMWH of the invention presents a degree of colouration greater than or equal to 6 in the range of colour reference solutions established in European Pharmacopoeia chapter 2.2.2. (method II), for at least 24 months, preferably for at least 36 months, at room temperature and 60 % relative humidity. This determination can be done following the method described in the European Pharmacopoeia (chapter 2.2.2; method II), or automatically using a colourimeter.

It has been observed that the LMWHs of the invention exhibit high stability. In particular, it has been observed that they are stable for at least 24 months, or even for at least 36 months at room temperature and 60 % relative humidity.

In one aspect, the invention relates to a method for obtaining low molecular weight heparins with an average molecular weight of between 3 and 3.8 KDa comprising the following steps:

a) preparing an aqueous solution of heparin sodium;

b) adding benzalkonium chloride to the solution of step a) to obtain benzalkonium heparinate;

c) dissolving the benzalkonium heparinate obtained in step b) in $CH_2CH_2$, adding Triton B and maintaining a temperature between 20 and 40ºC for 24 to 48 hours; and

d) carrying out at least two treatments with $H_2O_2$ of the depolymerized heparin obtained in step c) in a ratio of between 0.04 and 1.0 litres of $H_2O_2$ at 33 % w/v for each kg of depolymerized heparin in each treatment.

Particular and preferred embodiments for the low molecular weight heparin are as previously defined herein.

In a particular embodiment, the aqueous heparin sodium solution of step a) is prepared from heparin obtained from pig intestinal mucosa.

The addition of Triton B in step c) can be done by one or several sequential additions, for example by 1, 2, 3 or 4 sequential additions of Triton B.

In a specific embodiment, the addition of Triton B in step c) is performed in a maximum of three sequential additions, i.e. 1, 2 or 3, in each one of them adding Triton B in a weight ratio of 0.2:1 to 0.3:1 Triton B:benzalkonium heparinate. Preferably, the addition of Triton B in step c) is performed by three sequential additions of Triton B, and preferably each with the addition of Triton B in a weight ratio of 0.2:1 to 0.3:1 of Triton B:benzalkonium heparinate.

In one embodiment of the invention, the addition of Triton B in step c) is performed by three sequential additions of Triton B, such that after the first addition the reaction is maintained for 6-10 hours until the second addition, after the second addition the reaction is maintained for 12-20 hours until the third addition, and after the third addition the reaction is maintained for 6-10 hours. In a further embodiment, after the first addition the reaction is maintained for 7-9 hours until the second addition, after the second addition the reaction is maintained for 14-18 hours until the third addition, and after the third addition the reaction is maintained for 7-9 hours. In yet another embodiment, the reaction time after the first, second and third addition is about 8 hours, about 16 hours and about 8 hours, respectively.

In one embodiment, the temperature in step c) is between 25 and 35 ºC, preferably between 27 and 32 ºC.

In a preferred embodiment, the treatment with $H_2O_2$ in step d) is carried out on an aqueous solution of the depolymerized heparin.

Preferably, each treatment with $H_2O_2$ in stage d) is performed with a ratio of between 0.04 and 0.5 litres of $H_2O_2$ at 33 % w/v per kg of depolymerized heparin, preferably between 0.04 and 0.3 litres of $H_2O_2$ at 33 % w/v per kg of depolymerized heparin. In a particular embodiment, each treatment with $H_2O_2$ in stage d) is performed with a ratio between 0.04 and 0.2 litres of $H_2O_2$ at 33 % w/v per kg of depolymerized heparin. In a preferred embodiment, step d) comprises a first treatment with $H_2O_2$ of the depolymerized heparin obtained after step c) with a ratio between 0.05 and 0.25 litres of $H_2O_2$ at 33 % w/v per kg of depolymerized heparin, and a second treatment with $H_2O_2$ with a ratio of between 0.04 and 0.25 litres of $H_2O_2$ at 33 % w/v per kg of depolymerized heparin.

In another embodiment, step d) includes three treatments with $H_2O_2$.

Preferably, step d) is carried out at a temperature of between 20 and 50 ºC, preferably between 25 and 45 ºC.

Preferably, each treatment with $H_2O_2$ is performed for at least 3 hours. For example, for between 3 and 20 hours. In a particular embodiment, the first treatment with $H_2O_2$ is performed for a time of between 12 and 20 h, preferably between 14 and 18 h. Preferably, the second and subsequent treatments with $H_2O_2$ are performed for a time of between 3 and 7 h, preferably between 4 and 6 h.

In a preferred embodiment, step d) is carried out at a pH between 10.5 and 11.5. Additionally, the method of the invention may include an additional step of precipitation in methanol of the depolymerized heparin after each treatment with $H_2O_2$ in step d). In a particular embodiment, two treatments with $H_2O_2$ are carried out and a methanol precipitation step of the depolymerized heparin is carried out between the two treatments with $H_2O_2$. In another embodiment, three treatments with $H_2O_2$ are carried out and a methanol precipitation step of the depolymerized heparin is performed between the first and second treatments with $H_2O_2$ and between the second and third treatments with $H_2O_2$.

Preferably, after the first treatment with $H_2O_2$ in step d) the depolymerized heparin is precipitated in a solution of sodium acetate in methanol.

In a preferred embodiment, the LMWH obtained with the method of the invention is purified by precipitation with methanol.

The LMWH obtained can be subjected to lyophilization.

## BRIEF DESCRIPTION OF THE FIGURES

Figure 1. $^1$H NMR spectrum of LMWH obtained by the method of the invention with anomeric zone expansion.
Figure 2. Spectrum of $^1$H $^{13}$C HSQC of LMWH obtained by the method of the invention with anomeric zone expansion.

## EXAMPLES

The following specific examples are provided to illustrate the nature of the present invention. These examples are included for purposes of illustration only and should not be understood as limiting the invention claimed herein.

### Example 1

10 g of sodium heparin is dissolved in purified water and under agitation a 50 % (w/v) solution of benzalkonium chloride is added, forming benzalkonium heparinate. The product formed is washed several times with water to remove excess chlorides and finally the product is dried by lyophilization.

Benzalkonium heparinate is dissolved in methylene chloride and the temperature is adjusted to 30 $\pm$ 5 oC. Benzyltrimethylammonium hydroxide (Triton B) is added at 40 % w/v in methanol at a weight ratio of 0.25:1 Triton B:benzalkonium heparinate and allowed to react at the above temperature. The addition is repeated two more times, leaving the first addition of Triton B for 8 hours, the second for 16 hours and the third for another 8 hours. $H_2O_2$ at 33 % w/v is added to the solution of the depolymerized product, at a pH between 10.5 and 11.5; specifically, 0.1 $\pm$ 10 % litres of $H_2O_2$ at 33 % w/v are added per kg of benzalkonium heparinate and it is left to react at 30 $\pm$ 5oC for more than 3 hours (between 14 and 18 hours). After the reaction time, it is precipitated over a solution of sodium acetate in methanol and the crude low molecular weight heparin is isolated by centrifugation. This crude low molecular weight heparin is dissolved in water and precipitated again with methanol. The precipitate is dissolved in purified water and treated with $H_2O_2$ 33 % w/v; specifically, 0.08 $\pm$ 10 % litres of $H_2O_2$ 33 % w/v per kg of benzalkonium heparinate is added at a temperature of 40 $\pm$ 2 °C for more than 3 hours (about 5 hours). After the reaction period, the solution is neutralized and precipitated with methanol.

The precipitate is redissolved in purified water and treated again with 0.05 $\pm$ 10 % litres of $H_2O_2$ 33 % w/v per kg benzalkonium heparinate at a temperature of 40 °C $\pm$ 2 °C for more than 3 hours (about 5 hours). After the reaction period, the solution is neutralized and precipitated with methanol.

The concentration of benzalkonium heparinate in the solution in which both depolymerization and the first treatment with hydrogen peroxide are carried out is 20 % w/v $\pm$ 2 % in this and all other examples, unless otherwise indicated. The amount of hydrogen peroxide added at the different stages is equivalent to referencing both benzalkonium heparinate and depolymerized heparin, since after the different additions of Triton B the benzalkonium heparinate present in the reaction depolymerizes, giving rise to depolymerized heparin.

The purified product is dissolved in water and lyophilized, yielding 6.00 g of low molecular weight heparin with an average molecular weight of 3241 Da, an anti-FXa activity of 118 IU/mg and an anti-FIIa activity of 13.7 IU/mg and presents in the reducing terminal of its oligosaccharide chains a content of 1,6-anhydro residues between 1 and 15 % where the proportion of 1,6-anhydroglucosamine residues is greater or equal to that of 1,6-anhydromannosamine residues, unlike other low molecular weight heparins.

This low molecular weight heparin also has the advantage of being stable at room temperature for 24-36 months, presenting a degree of colouration $\geq$ 6 measured with a LICO® brand colourimeter that relates the colour according to the reference chromatic range established in Pharmacopoeia (Ph. Eur.(2.2.2, Method II)).

The degree of colouration, a parameter directly related to the stability of the product, made it possible to determine that the final packaged product was stable at room temperature for between 24 and 36 months.

### Example 2

Starting from 10 g of sodium heparin and repeating the steps indicated in example 1, 6.01 g of low molecular weight heparin with an average molecular weight of 3259 Da, an anti-FXa activity of 103 IU/mg and an anti-FIIa activity of 15.2 IU/mg are obtained. This low molecular weight heparin presents the characteristic of having in the reducing terminal end of its oligosaccharide chains a content of 1 ,6-anhydro residues between 1 and 15 %, where the proportion of 1,6-anhydroglucosamine residues is higher than that of 1,6-anhydromannosamine residues, unlike other low molecular weight heparins.

This low molecular weight heparin also has the advantage of being stable at room temperature for 24-36 months, presenting a colour grade $\geq$ 6 measured with a LICO® brand colourimeter that establishes the colour according to the reference chromatic range established in Pharmacopoeia (Ph. Eur.(2.2.2, Method II)).

The degree of colouration, a parameter directly related to the stability of the product, made it possible to determine that the final packaged product was stable at room temperature for between 24 and 36 months.

**Example 3**

10 g of sodium heparin is dissolved in purified water and under agitation a 50 % (w/v) solution of benzalkonium chloride is added, forming benzalkonium heparinate. The product formed is washed several times with water to remove excess chlorides and finally the product is dried by lyophilization.

Benzalkonium heparinate is dissolved in methylene chloride and the temperature is adjusted to 30 $\underline{o}$C $\pm$ 5 $\underline{o}$C. Benzyltrimethylammonium hydroxide (Triton B) is added at 40 % w/v in methanol at a weight ratio of 0.25:1 Triton B:benzalkonium heparinate and allowed to react at the above temperature. The addition is repeated two more times, leaving the first addition of Triton B for 8 hours, the second for 16 hours and the third for another 8 hours.

$H_2O_2$ at 33 % w/v is added to the solution of the depolymerized product, at a pH between 10.5 and 11.5; specifically, 0.1 $\pm$ 10 % litres of $H_2O_2$ at 33 % w/v/kg of benzalkonium heparinate and it is left to react at 30 $\pm$ 5 $\underline{o}$C for 16 hours. After the reaction time, it is precipitated over a solution of sodium acetate in methanol and the crude low molecular weight heparin is isolated by centrifugation.

This crude low molecular weight heparin is dissolved in water and precipitated again with methanol. The precipitate is dissolved in purified water and treated with $H_2O_2$ 33 % w/v; specifically, 0.08 $\pm$ 10 % litres of $H_2O_2$ 33 % w/v/ kg of benzalkonium heparinate is added at a temperature of 40 $\pm$ 2 °C. After 5 hours of reaction, the solution is neutralized and precipitated with methanol.

The purified product is dissolved in water and lyophilized, yielding 5.21 g of low molecular weight heparin with an average molecular weight of 3269 Da, an anti-FXa activity of 119 IU/mg and an anti-FIIa activity of 13.70 IU/mg and presents in the reducing terminal of its oligosaccharide chains a content of 1,6-anhydro residues between 1 and 15 %, where the proportion of 1,6-anhydroglucosamine residues is higher or equivalent to that of 1,6-anhydromannosamine residues, unlike other low molecular weight heparins.

This low molecular weight heparin also has the advantage of being stable at room temperature for 24-36 months, presenting a degree of colouration $\geq$ 6 measured with a LICO® brand colourimeter that relates the colour according to the reference chromatic range established in Pharmacopoeia (Ph. Eur.(2.2.2, Method II)).

The colour grade, a parameter directly related to the stability of the product, made it possible to determine that the final packaged product was stable at room temperature for between 24 and 36 months.

**Example 4**

Starting from 10 g of heparin sodium and repeating the steps indicated in Example 1, 6.25 g of low molecular weight heparin with an average molecular weight of 3172 Da, an anti-FXa activity of 108 IU/mg and an anti-FIIa activity of 13.1 IU/mg and a residue profile of 1,6-anhydro at the reducing terminus of its oligosaccharide chains between 1 and 15 %, where the proportion of 1,6-anhydroglucosamine residues is higher or equivalent to that of 1,6-anhydromannosamine residues, unlike other low molecular weight heparins such as enoxaparin.

This low molecular weight heparin also has the advantage of being stable at room temperature for 24-36 months, presenting a degree of colouration $\geq$ 6 measured with a LICO® brand colourimeter that relates the colour according to the reference chromatic range established in Pharmacopoeia (Ph. Eur. (2.2.2, Method II)).

The colour grade, a parameter directly related to the stability of the product, made it possible to determine that the final packaged product was stable at room temperature for between 24 and 36 months.

**Example 5**

Starting from 10 g of heparin sodium and repeating the steps indicated in Example 1, 6.34 g of low molecular weight heparin with an average molecular weight of 3347 Da, an anti-FXa activity of 110 IU/mg and an anti-FIIa activity of 14.7 IU/mg and a residue profile of 1,6-anhydro at the reducing terminus of its oligosaccharide chains between 1 and 15 %, where the proportion of 1,6-anhydroglucosamine residues is higher or equivalent to that of 1,6-anhydromannosamine residues, unlike other low molecular weight heparins such as enoxaparin.

This low molecular weight heparin also has the advantage of being stable at room temperature for 24-36 months, presenting a colour grade $\geq$ 6 measured with a LICO® brand colourimeter that establishes the colour according to the reference chromatic range established in Pharmacopoeia (Ph. Eur. (2.2.2, Method II)). The colour grade, a parameter directly related to the stability of the product, made it possible to determine that the final packaged product was stable at room temperature for between 24 and 36 months.

**Example 6**

Starting from 10 g of heparin sodium and repeating the steps indicated in Example 1, 7.11 g of low molecular weight heparin with an average molecular weight of 3400 Da, an anti-FXa activity of 119 IU/mg and an anti-FIIa activity of 15.0 IU/mg and a residue profile of 1,6-anhydro at the reducing terminus of its oligosaccharide chains between 1 and 15 %, where the proportion of 1,6-anhydroglucosamine residues is higher than that of 1,6-anhydromannosamine residues, unlike other low molecular weight heparins such as enoxaparin.

This low molecular weight heparin also has the advantage of being stable at room temperature for 24-36 months, presenting a degree of colouration ≥ 6 measured with a LICO® brand colourimeter that relates the colour according to the reference chromatic range established in Pharmacopoeia (Ph. Eur. (2.2.2, Method II)). The colour grade, a parameter directly related to the stability of the product, made it possible to determine that the final packaged product was stable at room temperature for between 24 and 36 months.

**Example 7**

Starting from 10 g of heparin sodium and repeating the steps indicated in Example 1, we obtain 6.92 g of low molecular weight heparin with an average molecular weight of 3328 Da, an anti-FXa activity of 117 IU/mg and an anti-FIIa activity of 14.9 IU/mg and a residue profile of 1,6-anhydro at the reducing terminus of its oligosaccharide chains between 1 and 15 %, where the proportion of 1,6-anhydroglucosamine residues is higher or equivalent to that of 1,6-anhydromannosamine residues, unlike other low molecular weight heparins such as enoxaparin.

This low molecular weight heparin also has the advantage of being stable at room temperature for 24-36 months, presenting a colour grade ≥ 6 measured with a LICO® brand colourimeter that establishes the colour according to the reference chromatic range established in Pharmacopoeia (Ph. Eur. (2.2.2, Method II)). The degree of colour, a parameter directly related to the stability of the product, made it possible to determine that the final packaged product was stable in an interval between 24 and 36 months.

**Example 8**

Starting from 10 g of heparin sodium and repeating the steps indicated in Example 3, 7.04 g of low molecular weight heparin with an average molecular weight of 3331 Da, an anti-FXa activity of 113 IU/mg and an anti-FIIa activity of 15.3 IU/mg and a residue profile of 1,6-anhydro at the reducing terminus of its oligosaccharide chains between 1 and 15 %, where the proportion of 1,6-anhydroglucosamine residues is higher or equivalent to that of 1,6-anhydromannosamine residues, unlike other low molecular weight heparins such as enoxaparin.

This low molecular weight heparin also has the advantage of being stable at room temperature for 24-36 months, presenting a colour grade ≥ 6 measured with a LICO® brand colourimeter that establishes the colour according to the reference chromatic range established in Pharmacopoeia (Ph. Eur.(2.2.2, Method II)). The colour grade, a parameter directly related to the stability of the product, made it possible to determine that the final packaged product was stable at room temperature for between 24 and 36 months.

**Example 9**

Starting from 10 g of heparin sodium and repeating the steps indicated in Example 3, 7.09 g of low molecular weight heparin with an average molecular weight of 3366 Da, an anti-FXa activity of 115 IU/mg and an anti-FIIa activity of 16.0 IU/mg and a residue profile of 1,6-anhydro at the reducing terminus of its oligosaccharide chains between 1 and 15 %, where the proportion of 1,6-anhydroglucosamine residues is higher or equivalent to that of 1,6-anhydromannosamine residues, unlike other low molecular weight heparins such as enoxaparin.

This low molecular weight heparin also has the advantage of being stable at room temperature for 24-36 months, presenting a colour grade ≥ 6 measured with a LICO® brand colourimeter that establishes the colour according to the reference chromatic range established in Pharmacopoeia (Ph. Eur.(2.2.2, Method II)). The colour grade, a parameter directly related to the stability of the product, made it possible to determine that the final packaged product was stable at room temperature for between 24 and 36 months.

**Comparative example 10 (no treatment with $H_2O_2$)**

10 g of sodium heparin is dissolved in purified water and under agitation a 50 % (w/v) solution of benzalkonium chloride is added, forming benzalkonium heparinate. The product formed is washed several times with water to remove excess chlorides and finally the product is dried by lyophilization.

Benzalkonium heparinate is dissolved in methylene chloride and the temperature is adjusted to 30 ± 5 ºC.

Benzyltrimethylammonium hydroxide (Triton B) is added at 40 % w/v in methanol at a weight ratio of 0.25:1 Triton B:benzalkonium heparinate and allowed to react at the above temperature. The addition is repeated two more times, leaving the first addition of Triton B for 8 hours, the second for 16 hours and the third for another 8 hours. The depolymerized product solution is precipitated over a solution of sodium acetate in methanol, and the crude low molecular weight heparin is isolated by centrifugation.

This crude low molecular weight heparin is dissolved in water and precipitated again with methanol. The precipitate is dissolved in purified water at a temperature of 40 ± 2 ºC at pH 11. After 5 hours, the solution is neutralized and precipitated with methanol.

The purified product is dissolved in water and lyophilized, yielding 5.28 g of low molecular weight heparin with an average molecular weight of 3280 Da, an anti-FXa activity of 112 IU/mg and an anti-FIIa activity of 13.70 IU/mg, and has a 1,6-anhydro residue content of 18.5 % at the reducing terminus of its oligosaccharide chains.

In addition, the ratio of 1,6-anhydroglucosamine residues to 1,6-anhydromannosamine residues is analysed; unlike in the previous examples, the 1,6-anhydromannosamine residues are larger than the 1,6-anhydroglucosamine residues.

We also analysed the degree of stability correlated with the colour of the sample, observing that from the tenth month at room temperature it presents a degree of colouration of 4 measured with a LICO® brand colourimeter that establishes the colour according to the chromatic range of reference established in Pharmacopoeia (Ph. Eur. (2.2.2, Method II)). The degree of colouration, a parameter directly related to product stability, made it possible to determine that the final packaged product was not stable for more than 9 months at room temperature, so the additions of $H_2O_2$ seem to be responsible for this behaviour.

**Comparative Example 11 (depolymerization at 60ºC)**

10 g of sodium heparin is dissolved in purified water and under agitation a 50 % (w/v) solution of benzalkonium chloride is added, forming benzalkonium heparinate. The product formed is washed several times with water to remove excess chlorides and finally the product is dried by lyophilization.

Benzalkonium heparinate is dissolved in methylene chloride and the temperature is adjusted to 60 ± 5 ºC. Benzyltrimethylammonium hydroxide (Triton B) is added at 40 % w/v in methanol at a weight ratio of 0.25:1 Triton B:benzalkonium heparinate and allowed to react at the above temperature. The addition is repeated two more times, leaving the first addition of Triton B for 8 hours, the second for 16 hours and the third for another 8 hours. Hydrogen peroxide at 33 % w/v is added to the dissolution of the depolymerized product at a pH between 10.5 and 11.5, specifically 0.1 ± 10 % litres of $H_2O_2$ at 33 % w/v /kg of benzalkonium heparinate and is left to react at 30 ± 5 ºC for 16 hours. After the reaction time, it is precipitated over a solution of sodium acetate in methanol and the crude low molecular weight heparin is isolated by centrifugation.

This crude low molecular weight heparin is dissolved in water and precipitated again with methanol. The precipitate is dissolved in purified water and treated with hydrogen peroxide at 33 % w/v; specifically, 0.08 ± 10 % litres of $H_2O_2$ 33 % w/v per kg of benzalkonium heparinate is added at a temperature of 40 ± 2 °C. After 5 hours of reaction, the solution is neutralized and precipitated with methanol.

The precipitate is dissolved in purified water and treated again with 0.05 ± 10 % litres of $H_2O_2$ 33 % w/v per kg of benzalkonium heparinate $H_2O_2$ 33 % w/v at a temperature of 40 °C ± 2 °C for about 5 hours. After the reaction period, the solution is neutralized and precipitated with methanol. The purified product is dissolved in water and lyophilized, yielding 5.10 g of low molecular weight heparin with an average molecular weight of 2341 Da, an anti-FXa activity of 109 IU/mg and an anti-FIIa activity of 2.8 IU/mg and presents in the reducing terminus of its oligosaccharide chains a 1,6-anhydro-residue content of 21%, where the proportion of 1,6-anhydroglucosamine residues is much lower than that of 1,6-anhydromannosamine residues.

We also analysed the degree of stability correlated with the colour of the sample, observing that from the tenth month at room temperature it presents a colour grade of 4 measured with a LICO® brand colourimeter that establishes the colour according to the chromatic range of reference established in Pharmacopoeia (Ph. Eur. (2.2.2, Method II)).

The degree of colouration, a parameter directly related to product stability, made it possible to determine that the final packaged product was not stable for more than 10-11 months at room temperature, requiring a cold chain for product stability to exceed 12 months.

**Example 12**

The products obtained in Examples 1 to 11 were analysed to determine the percentage of 1,6-anhydro residue content in a specific manner according to the method described in Enoxaparin Sodium monograph, 1097, European Pharmacopeia 9th Ed, described under Identification B, yielding the following results.

| Example | Average molecular weight, Da | 1,6-anhydrous content, % |
|---------|------------------------------|--------------------------|
| 1 | 3241 | 8 |
| 2 | 3259 | 9 |
| 3 | 3269 | 11 |
| 4 | 3172 | 8 |
| 5 | 3347 | 7 |
| 6 | 3400 | 5 |
| 7 | 3328 | 5 |
| 8 | 3331 | 4 |
| 9 | 3366 | 4 |
| 10 | 3280 | 18.5 |
| 11 | 2341 | 21 |

**Example 13**

The product obtained in example 1, is analysed by nuclear magnetic resonance, specifically by the $^1$H-RMN and $^1$H-$^{13}$C HSQC experiments.

$^1$H-RMN spectroscopy has been the most widely used technique for the study of these compounds since it is an abundant nucleus with a high gyromagnetic ratio. The region between 1.8 - 2.1 ppm comprises the signals corresponding to the N-acetyl groups or methyl groups of the reducing termini that can be synthetically included. The region between 2.8 - 4.6 ppm comprises the majority of the saccharide ring signals and has a high degree of overlap between them, making it difficult to extract structural information directly from this area. The signals corresponding to the anomeric protons are in the region between 4.6 - 6.0 ppm. Since this zone is much less populated with signals, it is possible to extract a great deal of information from it. Furthermore, in the case of LMWHs obtained by β-elimination mechanism, it also contains the signals corresponding to the H4 of the non-reducing termini of the molecule.

Specifically, the signals corresponding to the 1,6-anhdro residues appear at 5.57 and 5.62 ppm, corresponding to the anomeric proton of the 1,6-anhydromannosamine and 1,6-anhydroglucosamine structures, respectively, as described in the literature.

Another two-dimensional experiment of particular importance for the structural characterization of this type of compounds is $^1$H-$^{13}$C HSQC (Heteronuclear Single-Quantum Correlation), which correlates proton chemical shifts with carbon-13 chemical shifts and allows assigning the primary structures of oligosaccharides derived from GAGs and the monosaccharide composition.

The increased spectral dispersion achieved with this two-dimensional technique allows the quantification of the integrals of the overlapping signals in the corresponding one-dimensional spectra.

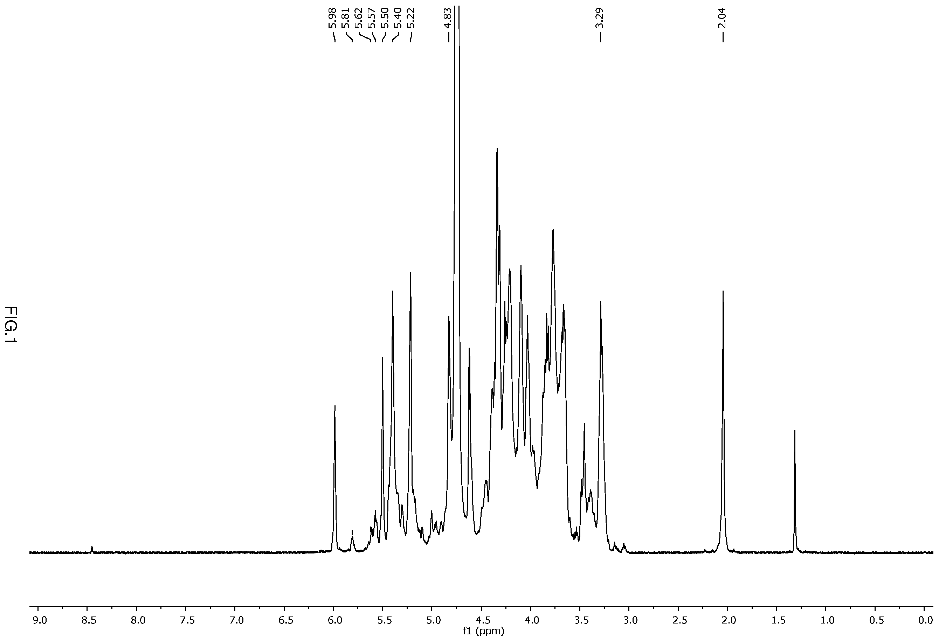

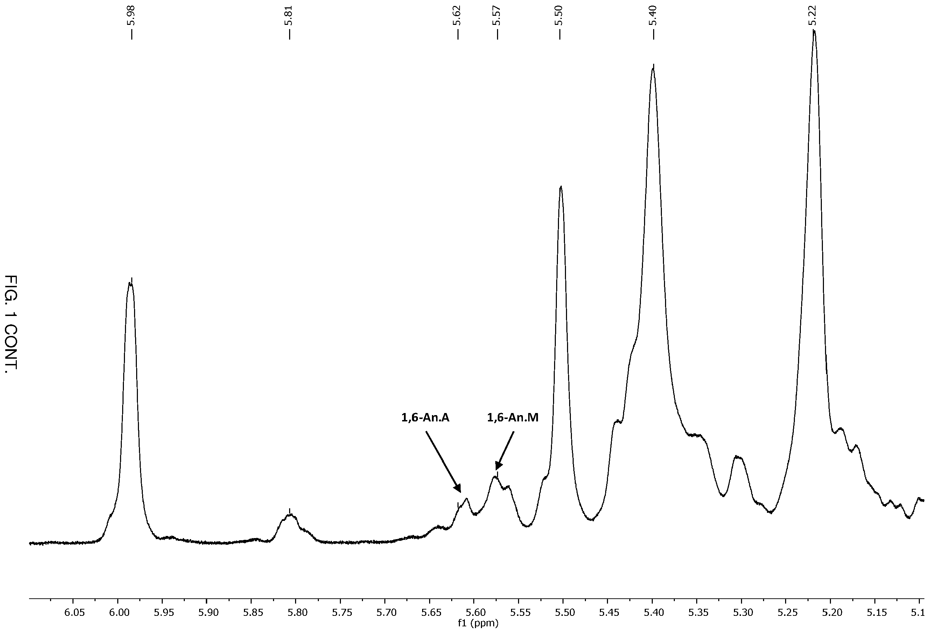

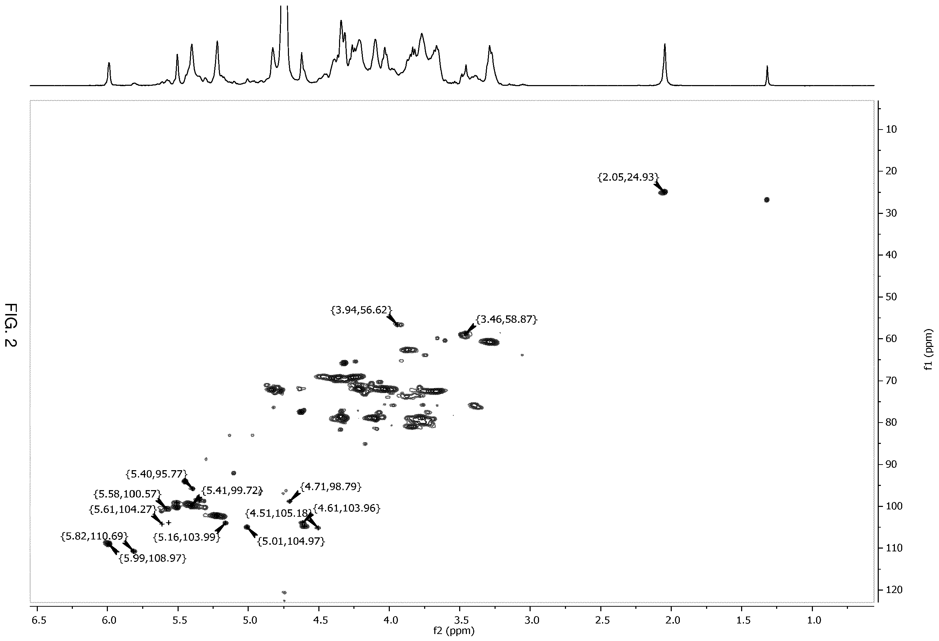

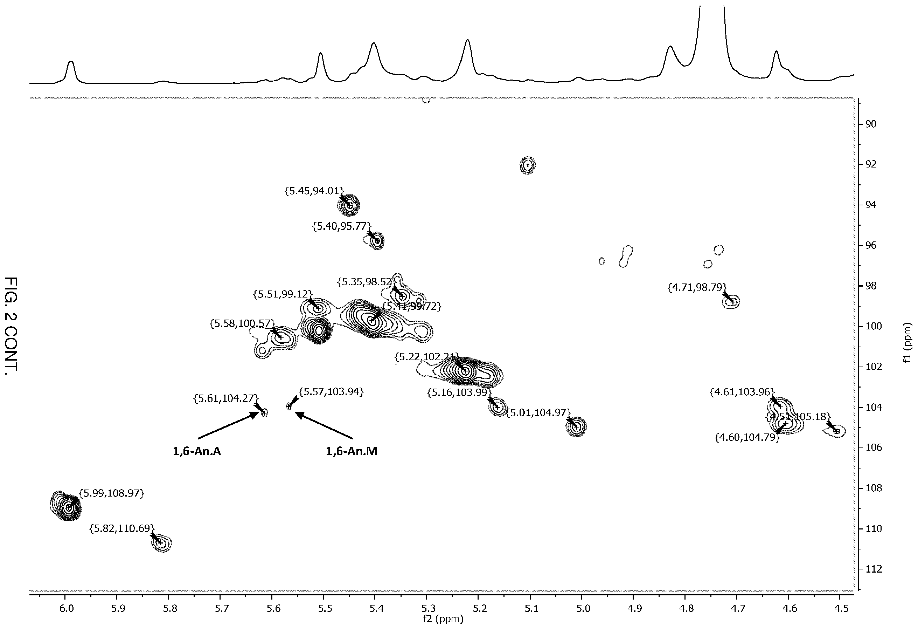

The signals corresponding to the 1,6-anhydro residues appear at 5.57-103.9 and 5.61-104.3 ppm, corresponding to the anomeric proton of the 1,6-anhydromannosamine and 1,6-anhydroglucosamine structures, respectively, as described in the literature and as observed in Figures 1 and 2 for the product sample obtained in Example 1.

**Example 14**

The products obtained in examples 1 to 11 were analysed for the residue content of 2-sulfo-amino-1,6-anhydro-2-deoxy-β-D-glucopyranose (1,6-anhydroglucosamine or 1,6-an.A) and 2-sulfo-amino-1,6-anhydro-2-deoxy-β-D-mannopyranose (1,6-anhydromannosamine or 1,6-an.M), according to the method known in the state of the art for $^1$H-$^{13}$C HSQC experiments, obtaining the following results.

| Example | 1,6-an.A, % mol. (relative ratio) | 1,6-an. M, % mol. (relative ratio) | Ratio 1.6-an.A / 1.6-an.M |
|---------|-----------------------------------|------------------------------------|---------------------------|
| 1 | 0.61 | 0.54 | 1.13 |
| 2 | 0.74 | 0.62 | 1.19 |
| 3 | 0.97 | 0.88 | 1.10 |

(continued)

| Example | 1,6-an.A, % mol. (relative ratio) | 1,6-an. M, % mol. (relative ratio) | Ratio 1.6-an.A / 1.6-an.M |
|---|---|---|---|
| 4 | 0.66 | 0.50 | 1.31 |
| 5 | 0.51 | 0.38 | 2.13 |
| 6 | 0.26 | 0.23 | 1.13 |
| 7 | 0.25 | 0.20 | 1.25 |
| 8 | 0.16 | 0.08 | 2.00 |
| 9 | 0.17 | 0.16 | 1.06 |
| 10 | 1.21 | 1.43 | 0.85 |
| 11 | 1.40 | 1.70 | 0.82 |

**Example 15: reproduction of example 1 of EP1070503 and of the same example with the addition of the hydrogen peroxide treatments**

Example 1 described in EP1070503 was reproduced as described therein and with a variant: adding after depolymerization with Triton B two stages of treatment with hydrogen peroxide at pH between 10.5 and 11.5, in the first stage adding 0.08 litres of $H_2O_2$ 33 % w/v per kg of benzalkonium heparinate at a temperature of 40 $\pm$ 2 ºC (5 hours of reaction) and in the second stage adding 0.05 litres of $H_2O_2$ 33 % w/v per kg of benzalkonium heparinate at a temperature of 40 ºC $\pm$ 2 ºC for about 5 hours.

By reproducing Example 1 of EP1070503, a low molecular weight heparin was obtained which presented at the reducing terminus of its oligosaccharide chains a 1,6-anhydro residue content of 0.3 %, whereas when the two hydrogen peroxide treatment steps described above were added this content was 6 %. Furthermore, the 1,6-anhydromannosamine residues are higher than the 1,6-anhydroglucosamine residues when reproducing Example 1 of EP1070503, while when performing the hydrogen peroxide treatments described above the molar ratio of the 1,6-anhydroglucosamine residues was higher or equal to that of the 1,6-anhydromannosamine residues.

When reproducing example 1 of EP1070503 and analysing the degree of stability correlated with the colour of the sample, it was observed that from the tenth month at room temperature it presents a degree of colouration of 4 measured with a LICO® brand colourimeter that relates the colour according to the reference chromatic range established in Pharmacopoeia (Ph. Eur. (2.2.2, Method II)). However, with the peroxide treatments described above, the product obtained proved to be stable at room temperature for 24-36 months presenting a degree of colouration $\geq$ 6 measured with a LICO® brand colourimeter that relates the colour according to the reference chromatic range established in Pharmacopoeia (Ph. Eur.(2.2.2, Method II)).

**Example 16:**

Examples 1 and 3 were repeated varying the amounts of hydrogen peroxide added, which always ranged in each treatment between 0.04 and 1.0 litres of $H_2O_2$ 33 % w/v /kg benzalkonium heparinate (or depolymerized heparin) and for times between 3 and 20 hours.

*litres of $H_2O_2$ 33 % w/v / kg benzalkonium heparinate

| First treatment* | Second treatment* | Third treatment* |
|---|---|---|
| 0.2 for 3 hours | 0.2 for 3 hours | - |
| 0.3 for 3 hours | 0.3 for 3 hours | - |
| 0.5 for 3 hours | 0.5 for 3 hours | - |
| 0.6 for 3 hours | 0.6 for 3 hours | - |
| 0.8 for 3 hours | 0.8 for 3 hours | - |
| 0.04 for 20 hours | 0.04 for 20 hours | 0.04 for 20 hours |
| 0.08 for 18 hours | 0.08 for 7 hours | 0.08 for 7 hours |
| 0.1 for 16 hours | 0.1 for 5 hours | 0.1 for 5 hours |

(continued)

| First treatment* | Second treatment* | Third treatment* |
|---|---|---|
| 0.25 for 14 hours | 0.25 for 4 hours | 0.25 for 4 hours |
| 0.1 for 16 hours | 0.08 for 5 hours | 0.05 for 5 hours |
| 0.04 for 18 hours | 0.05 for 7 hours | - |

In all cases, the heparins obtained had an average molecular weight of between 3 and 3.8 KDa, an anti-FXa activity of between 80-120 IU/mg and an anti-FIIa activity of between 5-20 IU/mg.

Likewise, in all cases the heparins obtained presented in the reducing terminus of their oligosaccharide chains a content of 1,6-anhydro residues between 1 and 15 % and the proportion of the 1,6-anhydroglucosamine residues was greater than or equal to that of the 1,6-anhydromannosamine residues.

**Claims**

1. Method for obtaining low molecular weight heparins with an average molecular weight between 3 and 3.8 KDa, comprising the following steps:

   a) preparing an aqueous solution of heparin sodium;
   b) adding benzalkonium chloride to the solution of step a) to obtain benzalkonium heparinate;
   c) dissolving the benzalkonium heparinate obtained in step b) in $Cl_2CH_2$, adding Triton B and maintaining a temperature between 20 and 40 ºC for 24 to 48 hours; and
   d) carrying out at least two treatments with $H_2O_2$ of the depolymerized heparin obtained in step c) in a ratio of between 0.04 and 1.0 litres of $H_2O_2$ at 33 % w/v for each kg of depolymerized heparin in each treatment.

   where stage d) is carried out at a pH between 10.5 and 11.5 and preferably for at least 3 hours for each treatment with $H_2O_2$.

2. Method according to claim 1, wherein the addition of Triton B of step c) is made in a maximum of three sequential additions, each addition of Triton B in a weight ratio of 0.2:1 to 0.3:1 Triton B:benzalkonium heparinate.

3. Method according to any one of claims 1 or 2, wherein the temperature of step c) is between 25 and 35 ºC, preferably between 27 and 32 ºC.

4. Method according to any one of claims 1 to 3, wherein step c) comprises three sequential additions of Triton B, such that after the first addition the reaction is maintained for 6-10 hours until the second addition, after the second addition the reaction is maintained for 12-20 hours until the third addition, and after the third addition the reaction is maintained for 6-10 hours.

5. Method according to any one of claims 1 to 4, wherein each treatment with $H_2O_2$ in step d) is performed with a ratio of between 0.04 and 0.5 litres of $H_2O_2$ at 33 % w/v per kg of depolymerized heparin, preferably between 0.04 and 0.3 litres of $H_2O_2$ at 33 % w/v per kg of depolymerized heparin.

6. Method according to any of claims 1 to 5, wherein stage d) is carried out at a temperature between 20 and 50 ºC, preferably between 25 and 45 ºC.

7. Method according to any one of claims 1 to 6, wherein step d) comprises a first treatment with $H_2O_2$ of the depolymerized heparin obtained after step c) with a ratio of between 0.05 to 0.25 litres of $H_2O_2$ at 33 % w/v per kg of depolymerized heparin, and a second treatment with $H_2O_2$ with a ratio of between 0.04 and 0.25 litres of $H_2O_2$ at 33 % w/v per kg of depolymerized heparin.

8. Method according to any one of claims 1 to 7, wherein the depolymerized heparin is precipitated in methanol between each treatment step with $H_2O_2$.

9. Method according to any one of claims 1 to 8, wherein the obtained low molecular weight heparin is purified by precipitation with methanol.

10. Method according to any one of claims 1 to 9, wherein the obtained low molecular weight heparin is subjected to lyophilization.

11. Low molecular weight heparin with an average molecular weight of between 3 and 3.8 kDa, obtained by the method according to any one of claims 1 to 10.

12. Low molecular weight heparin according to claim 11 **characterized in that** it has at the reducing terminus of its oligosaccharide chains a 1,6-anhydro residue content of between 1 and 15 %, preferably between 4 and 11 %.

13. Low molecular weight heparin according to any one of claims 11 or 12, wherein the molar ratio of the 1,6-anhydroglucosamine residues is greater than or equal to that of the 1,6-anhydromannosamine residues.

14. Low molecular weight heparin according to any one of claims 11 to 13, **characterized in that** it has an anti-FXa activity of between 80-120 IU/mg and an anti-FIIa activity of between 5-20 IU/mg.

15. Low molecular weight heparin according to any one of claims 11 to 14, wherein said heparin exhibits a degree of colouration greater than or equal to 6 in the range of colour reference solutions set forth in European Pharmacopoeia 2.2.2. method II, for at least 24 months, preferably for at least 36 months, at room temperature.

16. Low molecular weight heparin with an average molecular weight of between 3 and 3.8 kDa, **characterized in that** it has at the reducing terminus of its oligosaccharide chains a 1,6-anhydro residue content of between 1 and 15 %, preferably between 4 and 11 %.

17. Low molecular weight heparin according to claim 16, wherein the molar ratio of the 1,6-anhydroglucosamine residues is greater than or equal to that of the 1,6-anhydromannosamine residues.

18. Low molecular weight heparin according to any one of claims 16 to 17, **characterized in that** it has an anti-FXa activity of between 80-120 IU/mg and an anti-FIIa activity of between 5-20 IU/mg.

19. Low molecular weight heparin according to any one of claims 16 to 18, wherein said heparin exhibits a degree of colouration greater than or equal to 6 in the range of colour reference solutions set forth in European Pharmacopoeia 2.2.2. method II, for at least 24 months, preferably for at least 36 months, at room temperature.

FIG.1

FIG. 1 CONT.

FIG. 2

FIG. 2 CONT.

## INTERNATIONAL SEARCH REPORT

| International application No. |
|---|
| PCT/ES2020/070695 |

### A. CLASSIFICATION OF SUBJECT MATTER

*C08B37/10* (2006.01)

According to International Patent Classification (IPC) or to both national classification and IPC

### B. FIELDS SEARCHED

Minimum documentation searched (classification system followed by classification symbols)
C08B

Documentation searched other than minimum documentation to the extent that such documents are included in the fields searched

Electronic data base consulted during the international search (name of data base and, where practicable, search terms used)

EPODOC, INVENES, WPI, BIOSIS, MEDLINE/NLM, EMBASE/Elsevier, XPESP y Bases de Datos TXT

### C. DOCUMENTS CONSIDERED TO BE RELEVANT

| Category* | Citation of document, with indication, where appropriate, of the relevant passages | Relevant to claim No. |
|---|---|---|
| A | ES 2161615 A1 (ROVI LAB FARMACEUT SA) 01/12/2001, example 1 and claims 4 and 5 | 1-19 |
| A | CN 102399379 A (SHANGHAI SEANPHARM CO LTD) 04/04/2012, description and example 5 | 1-19 |
| A | EP 0040144 A1 (PHARMUKA LAB) 18/11/1981, the whole document. | 1-19 |
| A | ES 2003197 A6 (ROVI LAB FARMACEUT SA) 16/10/1988, the whole document. | 1-19 |
| A | EP 2881404 A1 (FARMACÉUTICOS ROVI S A LAB LABORATORIOS FARMACÉUTICOS ROVI S A) 10/06/2015, the whole document. | 1-19 |

☒ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 25/01/2021 | **(12/02/2021)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | M. García Coca  Telephone No. 91 3493411 |

Form PCT/ISA/210 (second sheet) (January 2015)

## INTERNATIONAL SEARCH REPORT

| | International application No. |
|---|---|
| | PCT/ES2020/070695 |

| C (continuation). | DOCUMENTS CONSIDERED TO BE RELEVANT | |
|---|---|---|
| Category * | Citation of documents, with indication, where appropriate, of the relevant passages | Relevant to claim No. |
| A | EP 2881404 A1 (FARMACÉUTICOS ROVI S A LAB LABORATORIOS FARMACÉUTICOS ROVI S A) 10/06/2015, the whole document. | 1-19 |
| A | LINHARDT ROBERT J et al.. "Production and chemical processing of low molecular weight heparins". Seminars In Thrombosis And Hemostasis 1999., 30/11/1998, Vol. 25, N° SUPPL. 3, pages 5-16, ISSN 0094-6176 the whole document. | 1-19 |

Form PCT/ISA/210 (continuation of second sheet) (January 2015)

**EP 4 144 766 A1**

## INTERNATIONAL SEARCH REPORT
Information on patent family members

| International application No. |
|---|
| PCT/ES2020/070695 |

| Patent document cited in the search report | Publication date | Patent family member(s) | Publication date |
|---|---|---|---|
| ES2003197 A6 | 16.10.1988 | EP0293539 A2 | 07.12.1988 |
| | | EP0293539 A3 | 12.04.1989 |
| | | DE3750036T T2 | 26.01.1995 |
| | | AT106901T T | 15.06.1994 |
| CN102399379 A | 04.04.2012 | CN102399379B B | 31.08.2016 |
| EP0040144 A1 | 18.11.1981 | ZA813176 B | 26.05.1982 |
| | | PT73024 A | 01.06.1981 |
| | | PT73024 B | 01.07.1982 |
| | | NZ197081 A | 31.07.1984 |
| | | NO156129B B | 16.11.1981 |
| | | NO156129C C | 29.07.1987 |
| | | MX6537E E | 04.07.1985 |
| | | MA19143 A1 | 31.12.1981 |
| | | KR830006333 A | 24.09.1983 |
| | | KR840001753B B1 | 19.10.1984 |
| | | JPS5710601 A | 20.01.1982 |
| | | JPS6051482B B2 | 14.11.1985 |
| | | IN152828 B | 14.04.1984 |
| | | IL62866 A | 29.06.1984 |
| | | IE51283 B1 | 14.11.1981 |
| | | IE811074L L | 14.11.1981 |
| | | HUT34769 A | 28.04.1985 |
| | | HU188667 B | 28.05.1986 |
| | | GR82283 B | 13.12.1984 |
| | | FR2482611 A1 | 20.11.1981 |
| | | FR2482611 B1 | 07.03.1986 |
| | | FI67865B B | 15.11.1981 |
| | | FI67865C C | 10.06.1985 |
| | | ES8206555 A1 | 16.08.1982 |
| | | DZ294 A1 | 13.09.2004 |
| | | DK211981 A | 15.11.1981 |
| | | DK168824B B1 | 20.06.1994 |
| | | CA1181744 A | 29.01.1985 |
| | | AU7051981 A | 19.11.1981 |
| | | AU535791B B2 | 05.04.1984 |
| | | ATA213681 A | 15.04.1987 |
| | | AT384428B B | 10.11.1987 |
| | | AR229510 A1 | 15.09.1983 |
| ES2161615 A1 | 01.12.2001 | US6384021 B1 | 07.05.2002 |
| | | PT1070503E E | 31.10.2005 |
| | | JP2001187802 A | 10.07.2001 |
| | | JP4897991B B2 | 14.03.2012 |
| | | ES2244165T T3 | 01.12.2005 |
| | | EP1070503 A1 | 24.01.2001 |
| | | EP1070503 B1 | 15.06.2005 |
| | | DK1070503T T3 | 05.12.2005 |
| | | DE69925821T T2 | 18.05.2006 |

Form PCT/ISA/210 (patent family annex) (January 2015)

## INTERNATIONAL SEARCH REPORT

Information on patent family members

International application No.

PCT/ES2020/070695

| Patent document cited in the search report | Publication date | Patent family member(s) | Publication date |
|---|---|---|---|
| | | BR9905820 A | 13.03.2001 |
| | | AT309693T T | 15.07.2005 |
| EP2881404 A1 | 10.06.2015 | HK1252082 A1 | 17.05.2019 |
| | | CY1120872T T1 | 11.12.2019 |
| | | SI2881404T T1 | 30.10.2018 |
| | | PL2881404T T3 | 31.08.2018 |
| | | HUE037251T T2 | 28.08.2018 |
| | | LT2881404T T | 10.08.2018 |
| | | HRP20180777T T1 | 10.08.2018 |
| | | PT2881404T T | 23.05.2018 |
| | | DK2881404T T3 | 06.06.2018 |
| | | CN108102007 A | 01.06.2018 |
| | | ES2669727T T3 | 29.05.2018 |
| | | BR112015002358 A2 | 04.07.2017 |
| | | HK1206042 A1 | 31.12.2015 |
| | | IN435MUMNP2015 A | 04.09.2015 |
| | | JP2015523451 A | 13.08.2015 |
| | | JP6298052B B2 | 20.03.2018 |
| | | CN104507972 A | 08.04.2015 |
| | | ES2445494 A1 | 03.03.2014 |
| | | ES2445494 B1 | 06.03.2015 |
| | | WO2014020227 A1 | 06.02.2014 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0040144 A
- EP 1070503 A
- EP 2881404 A

**Non-patent literature cited in the description**

- Heparins, Low-Molecular-Mass monograph, 0828. European Pharmacopeia
- European Pharmacopeia (Ph. Eur
- European Pharmacopeia
- European Pharmacopoeia (Ph. Eur.